# EUROPEAN PATENT SPECIFICATION

(11) **EP 3 788 939 B1**
(45) Date of publication and mention of the grant of the patent: **24.01.2024**
(21) Application number: 19195989.9
(22) Date of filing: 06.09.2019
(51) Int. Cl.: A61B 1/307, A61B 1/06, G02B 23/24, H04N 23/50, A61B 1/05, A61B 1/00

(54) **A TIP PART ASSEMBLY FOR AN ENDOSCOPE AND A METHOD OF MANUFACTURE OF A TIP PART ASSEMBLY OF AN ENDOSCOPE**
SPITZENTEILANORDNUNG FÜR EIN ENDOSKOP UND HERSTELLUNG EINER SPITZENTEILANORDNUNG FÜR EIN ENDOSKOP
ENSEMBLE DE PARTIE DE POINTE POUR ENDOSCOPE ET UN PROCEDE DE FABRICATION D'UN ENSEMBLE DE PARTIE DE POINTE POUR ENDOSCOPE

(43) Date of publication of application: 10.03.2021
(73) Proprietor: Ambu A/S, 2750 Ballerup (DK)
(72) Inventor: SØRENSEN, Morten, 2750 Ballerup (DK)
(74) Representative: AWA Denmark A/S

(56) References cited:
- JP-A- 2011 200 399
- JP-A- 2015 047 277
- US-A1- 2002 193 663
- US-A1- 2007 249 907
- US-A1- 2008 266 441
- US-A1- 2009 012 358
- US-A1- 2011 288 372

## Description

### Technical Field

The present disclosure relates to endoscopes and more specifically to a tip part assembly for an endoscope.

### Background

Endoscopes are well known for visually inspecting difficult to access places such as human body cavities. Typically, the endoscope comprises an elongated insertion tube with a handle at the proximal end, as seen from the operator, and visual inspection means, such as a built-in camera, at the distal end of the elongated insertion tube. This definition of the terms distal and proximal, i.e. proximal being the end closest to the operator and distal being the end remote from the operator, as used herein for endoscopes in general, is adhered to in the present specification.

As the name indicates, endoscopes are used for seeing inside things, such as lungs or other human body cavities of a patient. Modern endoscopes are therefore typically equipped with a light source and a vision receptor including a vision sensor, such as a camera or an image sensor. Provided that sufficient light is present, it is possible for the operator to see where the endoscope is steered and to set the target of interest once the tip has been advanced thereto. This therefore normally requires illumination of the area in front of the distal tip of the endoscope, in particular the field of vision of the camera(s). The light source, such as a light emitting diode, LED, or an optical fibre, may provide illumination.

Electrical wiring for the camera and other electronics, such as LED lighting accommodated in the tip part assembly at the distal end, run along the inside of the elongated insertion tube from the handle to the tip part assembly. Instead of using cameras, endoscopes may also be fibre-optic, in which case the optical fibres run along the inside of the elongated insertion tube to the tip part assembly. For some applications, a working or suction channel may run along the inside of the insertion tube from the handle to the tip part assembly, e.g. allowing liquid to be removed from the body cavity or allowing for insertion of surgical instruments or the like, into the body cavity. The suction channel may be connected to a suction connector, typically positioned at a handle at the proximal end of the insertion tube. For other applications, the working or suction channel may be omitted.

In order to be able to manoeuvre the endoscope inside the body cavity, the distal end of the endoscope may comprise a bending section with increased flexibility, e.g. an articulated tip part assembly allowing the operator to bend this section. Typically, this is done by tensioning or slacking steering wires also running along the inside of the elongated insertion tube from the articulated tip part assembly to a control mechanism of the handle.

A general desire in the field of endoscopy is to electrically insulate the insertion tube, and thus especially the tip part assembly, from the outside, so as to mitigate the risk of an insulation breakdown and a resulting excessive leakage current.

Another general desire in the field of endoscopy is to provide a tip part assembly which is liquid-sealed, so as to mitigate liquid ingress into the tip part assembly, and specifically into any electrical or optical components of the tip part assembly.

For some types of endoscopes, such as urethroscope, there is a desire to provide the tip part assembly of the endoscope with a smaller diameter or cross sectional extent, especially where the tip part assembly is to be inserted into narrower body cavities. In very narrow body cavities, even a reduction of 1 mm or less in the cross-sectional extent of a tip part assembly can have a noticeable effect on the comfort of the patient and may even make it possible to reach body areas not otherwise accessible. Providing a small size of the tip part assembly can especially be a challenge in cases where the endoscope comprises both a camera and a working channel extending through the tip part assembly since the camera and working channel are positioned one above the other within the tip part assembly, which takes up space in a radial direction of the tip part assembly.

US Pat. No. 9,125,582 discloses an endoscope similar to the endoscopes mentioned above, where the majority of the components of the endoscope's tip part assembly are embedded in a material of the housing of the tip part assembly.

US Pub. No. 2008/0132760 discloses a tip part assembly for an endoscope comprising a compartment at the distal end of the tip part assembly, for accommodating a filler such as an adhesive for securing a portion of various components such us a light source or vision receptor of the tip part assembly within the compartment.

US Pub. No. 2008/0266441 A1 relates to a solid-state image pickup device which photoelectrically converts a photographic light and thereby outputs avideo signal; and a lens barrel which, being approximately cylindrical in shape and being fastened to the solid-state image pickup device by a light-curing adhesive, holds a plurality of objective lenses, wherein the lens barrel has a tapered surface on a rear end face which opposes front part of the solid-state image pickup device, with distance between the tapered surface and the front part of the solid-state image pickup device increasing toward outer circumference.

US Pub. No. 2007/0249907 A1 relates to an imaging assembly for use in a medical imaging device such as an endoscope or the like. In one embodiment, the imaging assembly includes a transparent distal cap that is shaped to receive an image sensor insert. The image sensor insert has a cooling channel that supplies a cooling liquid or gas to one or more illumination sources.

US Pub. No. 2002/0193663 A1 relates to a hard distal component formed at the distal part of an inserting section and a leading bending piece included in a bending section are joined using a substantially cylindrical hard joint pipe. Build-in components including an imaging device that is a CCD are placed in the joint pipe. An adhesive is poured into the surroundings of the built-in components in the joint pipe, whereby the built-in components are locked. Thus, the structure of the distal component is simplified and the surroundings thereof are made thinner.

US Pub. No. 2009/0012358 A1 relates to an endoscope according to the present invention includes: an elongated insertion portion inserted in an examination object; an LED which is provided in a distal end portion of the insertion portion and is driven by supply of electric power; an image pickup portion which is provided in the distal end portion on a rear end side in the insertion direction than the LED, and which picks up an image of the examination object; a power supply line for power supply, a distal end in an insertion direction of which is positioned in the distal end portion on a rear end side in the insertion direction than the image pickup portion, and which is inserted in the insertion portion.

It is therefore desirable to provide a tip part assembly with a smaller outer diameter for an endoscope, such as an urethroscope, having electrically insulating properties and being mechanically stable.

### Summary

a) The invention is defined in claim 1 and relates to a method of manufacture of a tip part assembly for an endoscope..

In this way a tip part assembly of small dimensions, especially for urethroscopes, with an outer diameter of the circumferential wall of the housing of less than 3.3 mm or even smaller may be achieved. It has been realized that by providing a cup-shaped housing having an open proximal end and comprising a circumferential wall which together with a distal end wall encloses a spacing, a less complex housing structure may be achieved as the liquid adhesive may be filled into the spacing through the open proximal end of the housing, removing the need for providing a channel, port or the like in the housing for filling adhesive into the spacing. The less complex housing structure may allow the thickness of the circumferential wall of the housing to be reduced and so allow the outer diameter of the circumferential wall of the housing to be reduced. Therefore, the diameter or the cross-sectional extent of the housing and thus the tip part assembly can be reduced.

The tip part assembly may further comprise a bending section and the method comprise the steps:
f) providing the bending section, the bending section having a distal end segment, and
g) subsequent to step e), adjoining the distal end segment of the bending section and the proximal open end of the housing.

The camera assembly may further include a circuit board, the circuit board being positioned at the proximal end of the camera assembly.

The cup-shaped housing of the tip part assembly ensures that a minimum insulation thickness is present on all sides of the components housed within the spacing, this may improve or provide a more secure electrical insulation around the camera module, circuit board, image sensor and electrical connections.

The adhesive filled into the housing from the open proximal end thereof may provide greater robustness, mechanical stability and/or rigidity of the tip part assembly. A better attachment/fixation of the components within the housing, which may be of particular importance during operation of the endoscope where wires or cables may be pulled, and stress or pulling in the circuit board may occur, especially during bending of the bending section may also be achieved.

The term "endoscope" may be defined as a device suitable for examination of natural and/or artificial body openings, e.g. for exploration of a lung cavity. Additionally, or alternatively, the term "endoscope" may be defined as a medical device.

The term "cup-shaped" may be defined as the general hollow shape of a cup without a handle.

The circumferential wall may be an outer or exterior wall and may include an outer surface of the tip part assembly for facing the environment. Potentially, no parts of or only a sleeve of the tip part assembly are positioned outside an outer circumference of the circumferential wall.

In this specification, a proximal-distal direction may be defined as an axis extending along the parts of the insertion tube of the endoscope. Adhering to the definition of the terms distal and proximal, i.e. proximal being the end closest to the operator and distal being the end remote from the operator. The proximal-distal direction is not necessarily straight, for instance, if the insertion tube is bent, then the proximal-distal direction follows the curvature of the insertion tube. The proximal-distal direction may for instance be a centre line of the insertion tube.

The distal end of the tip part assembly may form a distal end of the endoscope.

The camera assembly may be a sub-assembly of the tip part and comprises a camera module which may include an image sensor, and a camera module housing in which the camera module may be arranged. Outer surfaces of the camera housing may be substantially box-shaped and/or parallelepipedal. The camera assembly may be fully embedded in the adhesive, potentially except for a distal lens thereof. The camera assembly may comprise at least one lens or a lens stack with two, three or more lenses. The lens stack may comprise a distal lens, i.e. the lens thereof positioned closest to the distal end of the tip part assembly. Similarly, the lens stack may comprise a proximal lens i.e. the lens positioned closest to the proximal end of the tip part assembly. The camera assembly may be at least partly housed in the housing.

The lens stack may be positioned distally of or in front of the image sensor. The camera module may further comprise a lens barrel which may hold and encase the lens stack. The lens stack may be stacked and/or the lens barrel may extend in the longitudinal direction.

The at least one lens, potentially the plurality of lenses, may be of one or more types chosen from the group consisting of: concave, convex, planoconcave, plano-convex, bi-convex, bi-concave.

The cup-shape of the cup-shaped housing is formed by the circumferential wall and the distal end wall enclosing a spacing where a proximal end of the housing is open and a distal end of the housing is closed. The housing may be tubular and/or cylindrical or substantially cylindrical and/or circular cylindrical or substantially circular cylindrical. The housing may provide electrical insulation and/or water tightness around the circuit board and electrical connections within the housing and may form a mould or container for adhesive and/or potting material poured or injected into the housing. The housing may ensure that a minimum insulation thickness is present on one or more sides or all outer surfaces of the tip part assembly. When an adhesive or a potting material is present in the housing, this may provide greater robustness, mechanical stability and/or rigidity of the tip part, and/or better attachment/fixation of components within the housing. This may be advantageous since wires or cables may be pulled during operation of the endoscope, pulling also in the circuit board, especially during bending of a bending section of the tip part assembly.

The housing may be an outer or exterior housing which may be exterior with respect to elements housed or enclosed therein, such as the camera assembly, wires, electrical components, LEDs, and the like.

The liquid adhesive may be of low dynamic viscosity, such as a dynamic viscosity below or equal to 200, 150, 120, 110 or 100 cP (centipoise, which is equal to mPa*s). This may ensure that the adhesive reaches all corners or substantially all corners of a free volume of the spacing.

The adhesive may be poured into the spacing through the open proximal end of the housing. The adhesive may be injected into the spacing through an injection needle or a nozzle inserted through or positioned above the said open proximal end of the housing.

A predefined amount of adhesive corresponding to a desired amount of adhesive in the spacing may be measured off during filling. Alternatively, or additionally, an upper or top level of the adhesive may be measured during filling to ensure a predefined amount of adhesive is filled into the housing.

The adhesive may be or may function as a potting material and may be unhardened or uncured when filling out the spacing. The adhesive may be cured after being filled into the spacing.

The adhesive may have electrically insulating properties and/or may be a potting material, e.g. such as disclosed in JP2011200399.

The adhesive and/or potting material may comprise or consist of or substantially consist of polyurethane adhesives, silicone adhesives, UV adhesives, resins, adhesive resins, thermosetting plastics, silicone rubber gels, polyurethane, silicone or combinations thereof.

The adhesive and/ or potting material may be heat cured, chemically cured, radiation cured (such as UV light cured) or moisture cured etc.

The bending section may comprise a number of hingedly interconnected segments including the distal end segment, a proximal end segment, and a plurality of intermediate segments positioned between the proximal end segment and the distal end segment. At least one hinge member may interconnect adjacent segments with each other. The bending section may be a section allowing the tip part assembly to bend relative to an insertion tube, potentially so as to allow an operator to manipulate the tip part assembly while inserted into a body cavity of a patient. The bending section may be moulded in one piece or may be constituted by a plurality of moulded pieces.

The bending section may or may not be attached to the housing by introducing a holder ring therebetween as is known in the art. A second adhesive (which may be the same material as the adhesive in the spacing) may be separately applied to attach the bending section end segment to the housing or, if a holder ring is present, to attach the distal end segment and the housing to the holder ring.

The tip part assembly may further include a working channel extending through the spacing of the housing to a hole or an opening in the end wall. The working channel may be moulded in one piece with the housing or may be provided separately from the housing. The working channel may extend into and/or through the distal end segment and/or the bending section.

The working channel may be tubular or substantially tubular and/or have a circumferentially extending, potentially cylindrical or circular cylindrical or substantially cylindrical or circular cylindrical, outer wall enclosing a working channel spacing. A material thickness of the tubular or substantially tubular wall may be less than or equal to 0.2 mm or less than or equal to ≤ 0.15 mm. The working channel may have an inner diameter of 0.8 to 2 mm or 1 to 1.6 mm or 1 to 1.4 mm. A wall thickness of a circumferential wall of the working channel may be 0.1 to 0.5 mm.

The working channel may comprise a chamfered portion, which may face at least a part of the camera assembly. The chamfered portion may, additionally or alternatively, be provided as a canted-off portion, a bevelled portion, or the like. The chamfered portion may be abutting at least part of the camera assembly or may be positioned with a distance to the camera assembly.

The chamfered portion may be part of the circumferential wall of the working channel, where a such is provided, or may be formed in one piece with the wall. The wall thickness of the chamfered portion of the circumferential wall may thus be smaller than along at least one other portion of the circumferential wall.

The working channel may allow liquid to be removed from a body cavity and/or allow insertion of surgical instruments or the like into the body cavity. The working channel may be provided as a channel extending from a proximal end of an endoscope to a distal end of the endoscope to guide a tool and/or to provide suction. A connector and/or a connecting portion may be provided at the proximal end of the endoscope to allow insertion of a tool into the working channel and/or to allow suction to be applied to the working channel. In some embodiments, the working channel comprises a built-in or integrated tool at or in the distal tip part assembly. Such a tool may be suitable for grabbing, taking, and/or holding elements in a part of a patient, in which the endoscope tip part is arranged during use.

The working channel may be at least partly housed in the housing. The working channel or a part thereof may be in one piece with the housing and/or may be provided as a separate part. The working channel may comprise a first portion potentially provided in one piece with the housing, and a second portion interconnected with the first portion. The second portion may be provided as a flexible tube and may be interconnected to the first portion by means of an adhesive. The working channel of the tip part assembly and the circumferential wall may be formed in one piece.

Especially if no working channel is present, the housing end wall and circumferential wall optionally do not comprise any holes or openings. If the tip part assembly includes a working channel, a working channel hole may be provided, potentially as the only or single hole or opening, in the end wall. Positioning a working channel in a hole in the end wall does not carry the same risk of adhesive flowing out at a periphery of the hole since adhesive residuals at a working channel distal end opening are not as disruptive as is the case for adhesive residuals at the camera assembly (and LEDs, cf. below).

The end wall may be formed integrally with or in one piece with the circumferential wall.

The tip part assembly may further include at least one light-emitting diode, LED, for illuminating a target, which may be positioned in the spacing before filling adhesive into the spacing. The LED(s) may be embedded or substantially embedded in the adhesive. The circuit board may include an arm leading to and connected to the LED. The circuit board may include an arm leading to and connecting to each at least one LED.

The tip part assembly may also include one or more light guides and/or LED lenses for guiding light from respective LED(s) to e.g. a front or distal end surface or end wall of the tip part assembly and/or a housing thereof. The light guide(s) may extend from the distal end of the tip part assembly to a respective LED or a respective set of LEDs. In some embodiments, the light guides are made from a transparent material. The light guide(s) may be moulded and/or may comprise a portion abutting the camera assembly and/or be arranged in front of the lens stack.

The LEDs may comprise a light emitting surface. The light emitting surface(s) may emit light in the proximal-distal direction. The light emitting surface(s) may be positioned in abutment with the housing, where this is provided, or in abutment with one or more light guides.

If no light guide is present, the LED may be positioned adjacent a proximal end surface of the distal end wall, in which case the adhesive may not be present between a front surface of the LED and a proximal end of the light guide, or between a front surface of the LED and a proximal surface of the end wall. The light guide may be at least partly embedded in the adhesive. The light guide may include a light shield, in particular on a surface of the light guide facing the camera assembly. The light shield may be provided as a layer of color or a material cladding with material having a low refraction index on said surface.

The method step of providing the housing may include the step of moulding the housing, which may occur before the assembly steps of the method. Moulding of the housing may occur as a two-component moulding in which two different materials are moulded in the same mould. For example, the end wall of the housing may be moulded in a first material, which may be transparent, and the circumferential wall may be moulded in a second, different material, which may be non-transparent and may include higher adhesive compatibility with the adhesive. Alternatively, the circumferential wall can be manufactured separately from the end wall. For example, the circumferential wall and the end wall can be moulded separately, or the end wall can be moulded, and the circumferential wall extruded. In this case, the circumferential wall and the end wall can be adhered to each other by means of an adhesive.

The term "higher adhesive compatibility" in the context of this specification, may be understood as a material providing a higher adhesive bond strength, being compatible with a wider range of adhesives, having a higher mechanical and/or chemical stability with adhesives.

The circuit board may be a printed circuit board (PCB) or a flexible printed circuit (FPC). The housing may be attached to the circuit board by means of the hardened adhesive. The entire or substantially the entire circuit board may be embedded in the adhesive.

FPCs may, throughout this specification, be a single- or double-sided flexible circuit or a rigid-flex circuit, and may comprise one or more layers of conductive material and two or more layers of insulating material, and/or may be a flexible flat cable having one or more conductors. In some embodiments, the FPC may be connected to a second flexible printed circuit and/or to a printed circuit board (PCB), e.g. comprising one or more cupper layers and one or more layers of insulating materials, such as layers of a FR-4 (flame retardant) composite material.

An electronic cable or electronic wires for connecting the circuit board to other parts of the endoscope may be at least partly embedded in the adhesive.

The camera assembly and potentially one or more LEDs may first be attached to one or more holders, where the interconnected holder(s) and camera assembly and potentially LED(s) are then positioned in the housing before filling in the adhesive.

The circuit boards and potentially wires for connecting the circuit board to other parts of the endoscope may be attached to the camera module and potentially the LEDs before or after positioning of the holder(s) and camera assembly and potentially the LED(s) in the housing.

The adhesive fills or substantially fills out all spacing volumes around the camera assembly, potentially except camera assembly holder(s) and/or a distal end or front surface of the camera assembly or a distal lens thereof. The adhesive may similarly fill out or substantially fill out all spacing volumes around the LED(s), potentially except LED holder(s) and/or a distal end or front surface of the LED(s). Alternatively, one or more holders may include one or more separation parts that may separate one or more separate spacing volumes so that the adhesive does not flow into these separate spacing volumes; this may be of advantage if it is desired to avoid that the adhesive flows in front of a camera assembly distal lens or an LED.

This may provide precise and repeatable positioning of the camera assembly and potentially the LEDs in the housing.

At least during step e) the housing may be positioned so that the proximal open end faces upwards. This may also be the case in step f).

This may allow easy filling of the adhesive into the housing as the filling of the adhesive may be aided by gravitational force.

An entire free volume of the spacing may be substantially filled up with the adhesive up until an upper adhesive level. Alternatively, an entire free volume of the spacing may be filled up until an upper adhesive level.

The adhesive may propagate to fill up also spacing volumes around the camera assembly and potentially the LED(s), even at a distal end of, i.e. in front of, the camera assembly and potentially the LED(s). With no empty spacings inside, the tip part assembly will be more rigid and less prone to being destroyed if forces are applied to it, e.g. if the tip part is accidentally dropped.

This may provide a robust and/or mechanically stable tip part assembly. It may also ensure that a correct and/or sufficient amount of adhesive is filled into the housing to attach the components therein to each other.

An outer diameter of the circumferential wall of the housing may be less than 3.3 mm.

In case the circumferential wall is not circular in cross section, this distance may instead be a largest cross-sectional extent of the circumferential wall. The outer diameter or largest cross-sectional extent of the circumferential wall of the housing may be less than 3.2, 3.1, 3.0. 2.9, 2.8, 2.7, 2.6 or 2.5 mm.

This may provide a tip part assembly with a small outer diameter and/or a tip part assembly with a small outer diameter of a circumscribed circle in a cross-section of the tip part assembly. This may improve comfort compared to endoscopes with tip part assemblies with larger outer diameters. It may also allow the tip part assembly to be used in applications which were previously inaccessible due to the outer diameter of tip part assemblies being too large.

A distal surface of a distal lens of the camera assembly may be positioned adjacent or abutting a proximal surface of the housing end wall.

This may provide increased security regarding tightness between the camera assembly, potentially a distal lens thereof, and the housing. Furthermore, the adhesive may tend to pour or flow out of the hole if the camera assembly, such as a distal lens thereof, is positioned in such a hole in the end wall. Adhesive spilling out here may flow to a surface of a distal surface of the camera assembly, potentially on or in front of a distal lens thereof, and distort the camera view in use.

Similarly, a distal and/or light emitting surface of an LED of the tip part assembly may be positioned adjacent or abutting a proximal surface of the housing. This proximal surface may be a proximal surface of the housing end wall or of a light guide. This may provide increased security regarding tightness between the LED and the housing. Furthermore, the adhesive may have a tendency to pour or flow out of the hole if the LED is positioned in such a hole in the end wall. Adhesive spilling out here may flow to a surface of a distal or front surface of the LED and distort light from the LED.

For both the camera assembly and LED, if no corresponding hole is provided in the end wall, and the camera assembly, potentially a distal lens thereof, and/or LED(s) is/are instead positioned with a distal surface adjacent to a proximal surface of the housing as described above, a layer of adhesive may be present between the distal lens or LED and the proximal surface of the housing. This layer of adhesive may be well-defined or have a uniform or substantially uniform thickness along the adjacent surfaces. This may minimize distortion of light shining through the layer of adhesive. Alternatively, the camera assembly, potentially a distal lens thereof, or LED distal surface abuts the housing proximal wall so that no or substantially no adhesive is present therebetween. A low viscosity adhesive as disclosed herein may be advantageous also to provide a thin layer of glue in this context. The layer of glue may be injected first directed against a volume between the distal and proximal surfaces after which the remaining adhesive is filled into the housing. Alternatively, not falling within the scope of the claims, the end wall comprises a hole for a distal lens of the camera assembly and/or an LED and/or an LED light guide, which is then positioned in the hole upon positioning of the camera assembly in the spacing.

The circumferential wall and/or the end wall of the housing may be transparent.

In this context, transparent is meant to describe that light or a suitable amount of light according to the purpose, i.e. light into the camera assembly and/or light from LED(s), is able to pass through the wall.

The housing may comprise or consist or substantially consist of a transparent plastic or plastic polymer material, such as: a polycarbonate (PC) polymethylmethacrylate (PMMA), Polyethylene Terephthalate (PET), Amorphous Copolyester (PETG), Polyvinyl Chloride (PVC), Liquid Silicone Rubber (LSR), Polyethylene (PE), Fluorinated Ethylene Propylene (FEP), Styrene Methyl Methacrylate (SMMA), Styrene Acrylonitrile Resin (SAN) and/or Methyl Methacrylate Acrylonitrile Butadiene Styrene (MABS). This may remove the need for a window or opening for the camera module and/or LED(s) in the circumferential wall and/or the end wall to allow the camera assembly to register a target area of the endoscope and/or for light emitted from the LED(s) to illuminate a target area of the endoscope. This again may provide a housing that is more resistant to ingress of liquid compared to a housing with an opening in the circumferential wall and/or end wall. Similarly, improved electrical insulation of the components in the housing may be achieved compared to a housing with an opening in the circumferential wall and/or end wall. Compared to a tip part assembly with holes, openings, and/or windows in the circumferential wall and/or end wall, it may provide a tip part assembly that is easier to manufacture as adhesive may not run or spill out of the holes, openings, and/or windows.

The term "target" or "target area" in the context of this specification, may be understood as an object or area of interest that the tip part assembly of the endoscope is being used to analyze.

The tip part assembly may further include one or more light guides and/or LED lenses. The tip part assembly may further include one or more light guides and/or LED lenses in one piece with housing. One or more of these light guides and/or LED lenses may be moulded in one piece with the housing; in that case, at least these parts of the housing may be transparent.

This may provide unobstructed and well-defined guidance of light emitted by the LED(s) towards a target area.

Step c) may include moulding the housing in a two-component moulding process, wherein the end wall of the housing is moulded in a first material, which is transparent, and the circumferential wall is moulded in a second, different material, which is non-transparent.

This may allow the camera assembly to register a target area through the transparent end wall whilst the circumferential wall with higher adhesive compatibility allows secure adhesive attachment of the camera assembly to the housing. This may also remove the need for an opening or window for the camera assembly or LED(s) in the end wall or circumferential wall.

Similarly, it may allow an LED to illuminate a target area through the transparent end wall, whilst the circumferential wall with higher adhesive compatibility allows secure adhesive attachment of the LED to the housing.

The second material may include a higher adhesive compatibility.

A wall thickness of the circumferential wall may increase from the proximal end of the housing towards the distal end of the housing.

Hereby, a more secure electrical insulation and/or mechanical stability may be provided since the wall thickness may be provided to be larger in an area of the endoscope that may be most exposed or stressed during use.

Furthermore, a tip part assembly for an endoscope according to claim 11 is provided.

This may provide a tip part assembly with a small outer diameter and/or a tip part assembly with a small outer diameter of a circumscribed circle in a cross-section of the tip part assembly. It may further provide a robust tip part assembly.

The tip part assembly may further comprise:
- a bending section having a distal end segment, and
- wherein the distal end of the bending section and the proximal open end of the housing are adjoined to each other.

The camera assembly may further include a circuit board, the circuit board being positioned at the proximal end of the camera assembly.

Any features of the embodiments of the method according to this disclosure may also be present in the tip part according to this disclosure.

An entire volume of the spacing not preoccupied by the camera assembly and potential further components positioned in the spacing may be substantially filled up with the adhesive up until an upper adhesive level, the upper adhesive level may be a level of the adhesive towards the proximal end of the tip part assembly.

Alternatively, an entire volume of the spacing not preoccupied by the camera assembly and potential further components positioned in the spacing is filled up with the adhesive up until an upper adhesive level, the upper adhesive level being a level of the adhesive towards the proximal end of the tip part assembly.

Alternatively, this could be defined as there substantially not being any air pockets present within the spacing up until an upper adhesive level.

Alternatively, this could be defined as there not being any air pockets present within the spacing up until an upper adhesive level.

The adhesive may propagate to fill up also spacing volumes around the camera assembly and potentially the LED(s), even at a distal end of, i.e. in front of, the camera assembly and potentially the LED(s). With no empty spacings inside, the tip part assembly will be more rigid and less prone to being destroyed if forces are applied to it. E.g. if the tip part is accidentally dropped.

"Provided separately from" may include or may mean that the adhesive and the housing are not integral or are not in one piece or are not moulded in one piece with each other.

If the housing is positioned upright with the housing proximal end facing upwards, the adhesive level is the level that the adhesive reaches in an upward direction.

An outer diameter of the circumferential wall of the housing may be less than 3.3 mm.

In case the circumferential wall is not circular in cross section, this distance may instead be a largest cross sectional extent of the circumferential wall. The outer diameter or largest cross sectional extent of the circumferential wall of the housing may be less than 3.2, 3.1, 3.0. 2.9, 2.8, 2.7, 2.6 or 2.5 mm. This may allow the endoscope to be used in places otherwise not accessible due to size limitations.

A distal surface of a distal lens of the camera assembly is positioned abutting a proximal surface of the housing end wall.

The circumferential wall and/or the end wall of the housing may be transparent.

An aspect of this disclosure relates to an endoscope comprising a tip part assembly manufactured according to claim 1 or a tip part assembly according to claim 11.

The endoscope may comprise a control element. The control element may be configured to allow an operator to control a tip part assembly of the insertion tube by at least one steering wire. The control element may allow bending the tip part assembly in at least one direction, potentially in two directions, the two directions potentially being opposite. The control element may be accommodated in an operating handle. The control element may include a lever allowing an operator to control the control element. The lever may extend outwardly from the control element, potentially through an operating handle. The control element may be in the form of a roller or a roller disc.

The endoscope may comprise an operating handle. The operating handle may be suitable for allowing an operator to grip and to operate the endoscope, potentially with one hand. The operating handle may comprise a handle housing arranged at a proximal end of the insertion tube. The handle housing may accommodate the control element.

The insertion tube and/or a distal end thereof and/or the tip part assembly thereof may be suitable for insertion into a body cavity, potentially a kidney, through a body opening, potentially a urinary passage or a urethra. The body may be a natural and/or artificial body, potentially a human body. The insertion tube may extend from the operating handle towards a distal end of the endoscope.

Additionally or alternatively, the endoscope may form part of a system for visually inspecting inaccessible places such as human body cavities, the system further comprising a monitor. The endoscope may be connectable to the monitor, and the monitor may allow an operator to view an image captured by the camera assembly of the endoscope.

A person skilled in the art will appreciate that any one or more of the above aspects of this disclosure and embodiments thereof may be combined with any one or more of the other aspects of the disclosure and embodiments thereof.

### Brief description of drawings

The tip part assemblies and methods will now be described in greater detail based on non-limiting exemplary embodiments and with reference to the drawings, on which:
FIG. 1a shows a perspective view of an endoscope in which a tip part assembly according to the present disclosure is implemented,
FIG. 1b shows a perspective view of a monitor to which the endoscope of FIG. 1a is connected,
FIG. 2 shows a plane view of a bending section of the endoscope of FIG. 1a and FIG. 1b,
FIG. 3 shows a perspective view of the camera assembly of a tip part assembly of FIG. 1a and 1b,
FIG. 4a shows a perspective view of a first embodiment of a tip part assembly of FIG. 1a and 1b, falling under the scope of the claims,
FIG. 4b shows a perspective view of a second embodiment of a tip part assembly of FIG. 1a and 1b, falling under the scope of the claims,
FIG. 4c shows a perspective view of a third embodiment of a tip part assembly of FIG. 1a and 1b, not falling under the scope of the claims,
FIG. 4d shows a view onto the distal end of the tip part assembly of FIG. 4b,
FIG. 4e shows a section view A-A of the tip part assembly of FIG. 4d,
FIG. 4f shows a section view of the tip part assembly of FIG. 4d filled with an adhesive,
FIG. 5a shows a perspective view of a LED holder with LEDs,
FIG. 5b shows a perspective view of a camera holder and light guide,

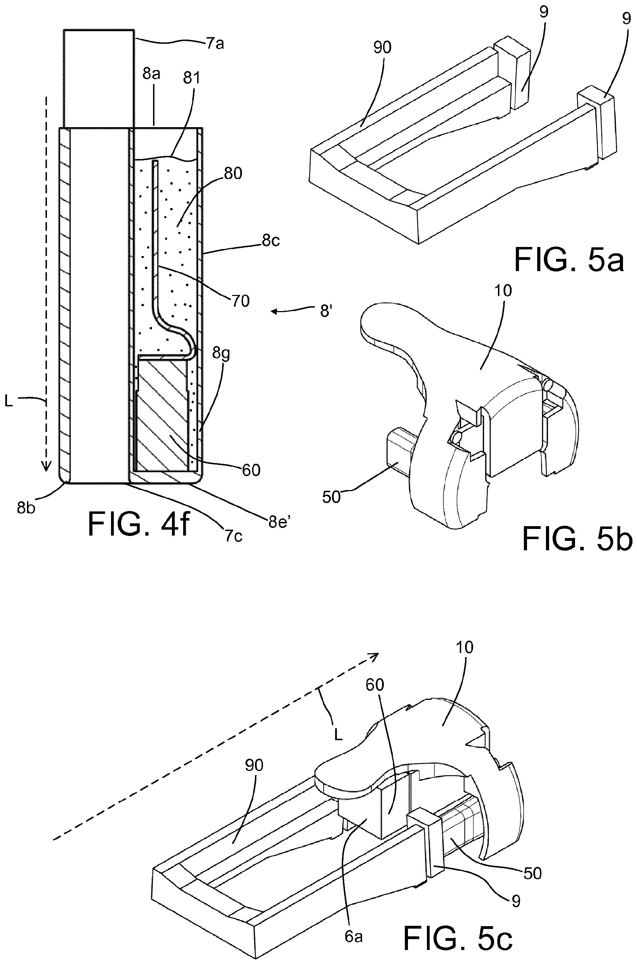

FIG. 5 shows a perspective view of an assembly of camera module in camera holder, LEDs in LED holder and light guides.

### Detailed description

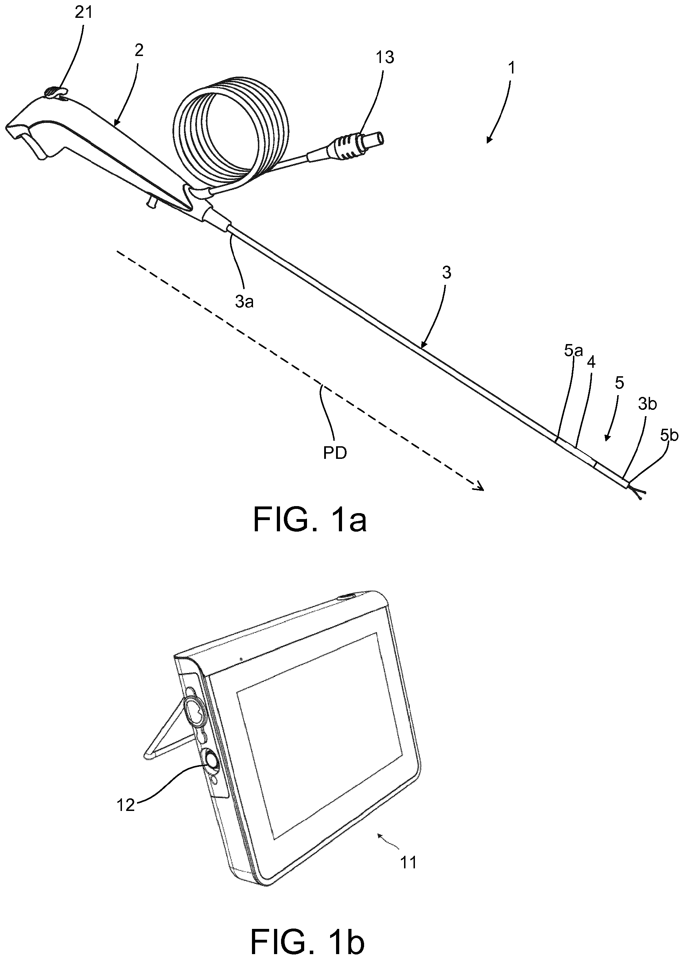

Referring first to FIG. 1a, an endoscope 1 is shown. The endoscope is disposable, and not intended to be cleaned and reused. The endoscope 1 comprises an elongated insertion tube 3. At a proximal end 3a of an insertion tube 3 of the endoscope 1, an operating handle 2 is arranged. The operating handle 2 has a control lever 21 for manoeuvring a tip part assembly 5 at a distal end 3b of the insertion tube 3 by means of a steering wire and bending section 4. A camera assembly 6 is positioned in the tip part assembly 5 and is configured to transmit an image signal through a monitor cable 13 of the endoscope 1 to a monitor 11.

The tip part assembly 5 has a proximal end 5a for being connected to other parts of the endoscope 1, and a distal end 5b positioned oppositely from the proximal end 5a forming the distal end 3b of the endoscope 1.

In Fig. 1b, a monitor 11 is shown. The monitor 11 may allow an operator to view an image captured by the camera assembly 6 of the endoscope 1. The monitor 11 comprises a cable socket 12 to which a monitor cable 13 of the endoscope 1 can be connected to establish a signal communication between the camera assembly 6 of the endoscope 1 and the monitor 11.

The proximal-distal direction PD is a direction extending along the parts of the insertion tube 3 of the endoscope 1.

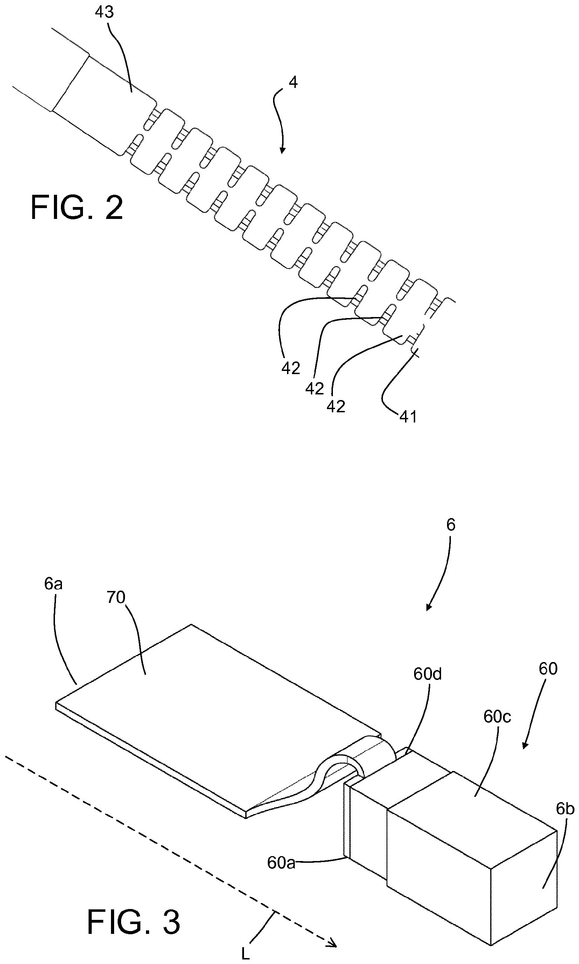

Referring to FIGs. 3 - 4f, the tip part assembly 5 is manufactured by providing a bending section 4, camera assembly 6 and cup-shaped housing 8, 8', 8" comprising a circumferential wall 8g and distal end wall 8e, 8e', 8e" enclosing a spacing as disclosed herein and inserting the camera assembly 6 through the open proximal end 8a of the housing 8, 8', 8" so that the camera assembly 6 is at least partly positioned within the spacing. Subsequent to inserting the camera assembly 6, the spacing is filled with a liquid adhesive 80 through the open proximal end 8a of the housing 8, 8', 8" so that the camera assembly 6 is at least partly embedded in the adhesive 80. The adhesive 80 is then allowed to harden whereby the adhesive 80 attaches the housing 8, 8', 8" and the camera assembly 6 whereafter the distal end segment 41 of the bending section 4 and the proximal open end 8a of the housing 8, 8', 8" are adjoined.

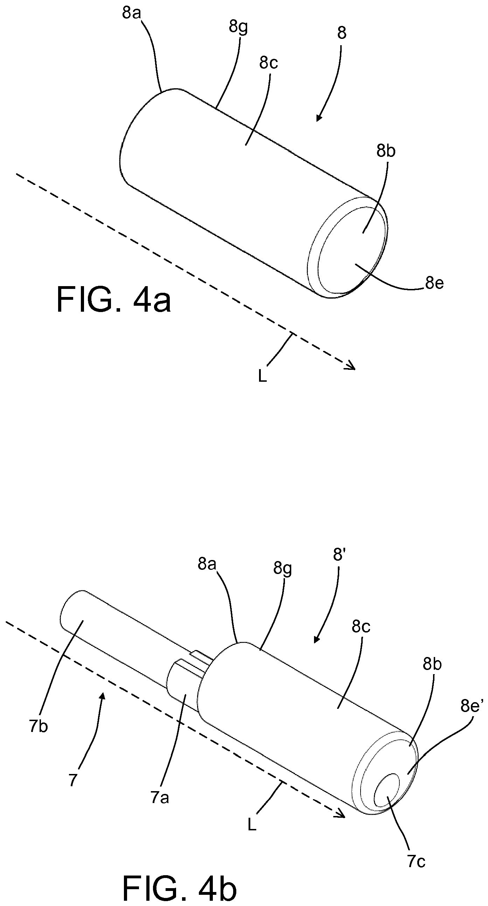

Turning to FIG. 2, a plane view of the bending section 4 is provided. The bending section 4 allows the tip part assembly 5 to bend relative to the insertion tube 3, so as to allow an operator to manipulate the tip part assembly 5 while inserted into a body cavity of a patient. The bending section 4 is moulded in one piece, but may alternatively be constituted by a plurality of moulded pieces. The bending section 4 comprises a number of hingedly connected segments including a distal end segment 41, a proximal end segment 43, and a plurality of intermediate segments 42 positioned between the distal end segment 41 and the proximal segment 43. The distal end segment 41 is adapted for being connected and/or attached to a housing of a tip part assembly, such as the housing 8, 8', 8" of FIGs. 4a - 4f at a proximal end 8a of the housing 8, 8', 8". A second adhesive (which may be the same material as the adhesive 80 in the spacing) is separately applied to adjoin the bending section end segment 41 to the housing 8, 8', 8".

FIG. 3 shows a perspective view of the camera assembly 6 which is a sub-assembly of the tip part assembly 5 and comprises camera module 60 a circuit board 70 and a camera module housing 60c which house the camera module 60. The camera module 60 includes an image sensor (not shown) and a lens stack (not shown. The camera module 60 extends in the longitudinal direction L. The circuit board 70 is electrically connected to the camera module 60 through a proximal connection surface 60d at a proximal end 60a of the camera module 60. The circuit board 70 is a flexible printed circuit (FPC). Alternatively, the circuit board 70 could be a printed circuit board (PCB).

The outer surfaces of the camera module housing 60c are substantially box-sha ped.

The lens stack also comprises a proximal lens (not shown) i.e. the lens positioned closest to the proximal end 5a of the tip part assembly 5. The camera assembly 6 is housed in the housing 8.

Turning to FIGs 4a - 4f, the cup-shaped housing 8, 8', 8" of the first, second and third embodiment of the tip part assembly are tubular and substantially cylindrical and made of polycarbonate. The outer diameter of the circumferential wall 8g of the housing 8, 8', 8" is 3.2 mm. The housing 8, 8' and 8" has a circumferentially extending outer surface 8c for facing the environment. The outer surface 8c encloses a volume and extends in the longitudinal direction L between a proximal end 8a and a distal end 8b of the housing 8, 8', 8".

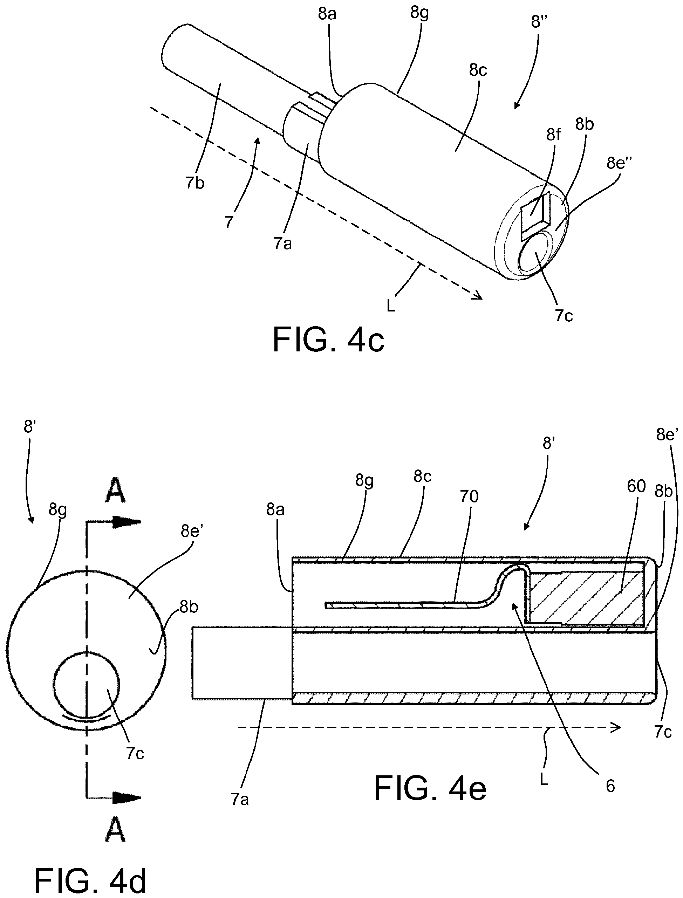

Where the housing 8, 8' does not comprise a hole, a window, or an opening 8f for the camera assembly 6, such as in the first and second embodiments in FIGs 4a, 4b, 4d, 4e and 4f or a distal lens thereof (not shown), the camera assembly 6 is positioned in the housing 8, 8' with its distal end 6b adjacent to the end wall 8e, 8e' of the housing 8, 8'. The housing end wall 8e, 8e' is transparent and allows the vision sensor (not shown) of the camera assembly 6 to register an area the endoscope 1 is targeting.

Where the housing comprises a hole, a window, or an opening 8f for the camera assembly 6 or a distal lens thereof, such as the third embodiment as seen FIG. 4c, the distal end 6b of the camera assembly 6 is positioned adjacent the window 8f.

The housing 8, 8', 8" provides electrical insulation and water tightness around the circuit board 70 and electrical connections within the housing 8, 8', 8" and forms a mould or container for adhesive 80 poured into the housing 8, 8', 8". The housing 8, 8', 8" ensures that a minimum insulation thickness is present on all outer surfaces of the tip part assembly 5. The liquid adhesive 80 present in the housing 8, 8', 8", as seen in FIG. 4f, provides greater robustness, mechanical stability and rigidity of the tip part assembly, and better attachment of components within the housing 8, 8', 8". The camera assembly 6 is substantially fully embedded in the adhesive 80, except for a distal lens (not shown) thereof i.e. the lens positioned closest to the distal end 5b of the tip part assembly 5. The distal surface of the distal lens (not shown) of the camera assembly 6 is positioned adjacent a proximal surface of the housing end wall 8e, 8e'.

The liquid adhesive 80 has electrically insulating properties and is of a low dynamic viscosity below or equal to 200cP. This helps ensure that the adhesive reaches substantially all corners of a free volume of the spacing.

In the first embodiment of the tip part assembly, a predefined amount of adhesive 80 corresponding to a desired amount of adhesive 80 in the spacing is measured off during filling. In the second embodiment an upper level 81 of the adhesive 80 is measured during filling to ensure a predefined amount of adhesive 80 is filled into the housing 8, 8', 8". The adhesive 80 also functions as a potting material and is unhardened and uncured when filling out the spacing. After the adhesive 80 has been filled into the spacing it is hardened and cured.

The housing 8, 8', 8" is an outer housing which is exterior with respect to elements housed or enclosed therein, such as the camera assembly 6, wires, electrical components, LEDs, and the like.

The method step of providing the housing 8, 8', 8" includes the step of moulding the housing 8, 8', 8", which occurs before the assembly steps of the method. Moulding of the housing 8, 8', 8" occurs as a two-component moulding in which two different materials are moulded in the same mould. The end wall 8e, 8e', 8e" of the housing 8, 8', 8" is moulded in a first material, which is transparent, and the circumferential wall 8g is moulded in a second, different material, which is non-transparent and includes a higher adhesive compatibility with the adhesive 80. That is, the end wall 8e, 8e', 8e" is formed in one piece with the circumferential wall 8g.

To improve the electrical insulation in the area around the circuit board 70 and a connection thereof to the camera assembly 6 in the housing 8, 8', 8" the wall thickness of the circumferential wall 8g increases from the distal end 8b of the housing 8, 8', 8" towards the proximal end 8a of the housing 8, 8', 8".

In the second and third embodiment shown in FIGs. 4b - 4f the first portion 7a of the working channel 7 is housed in the housing 8', 8" and comprises an opening 7c in a distal surface 8e', 8e" of the housing 8', 8"

Referring to FIGs 4b - 4f, the working channel 7 has a first portion 7a with a circumferentially extending circular cylindrical outer wall enclosing a working channel spacing. The working channel 7 further comprises a second portion 7b. The second portion is connected liquid tight to the first portion 7a by an adhesive. The working channel second portion 7b extends along the proximal-distal direction PD through the insertion tube 3 to the proximal end 3a of the insertion tube 3. The second portion 7b is provided as a flexible tube.

The working channel 7 allows liquid to be removed from a body cavity or allows insertion of surgical instruments or the like into the body cavity. The working channel 7 extends from a proximal end of an endoscope to a distal end of the endoscope 1.

The working channel 7 has an inner diameter of 1.2 mm. A wall thickness of a circumferential wall of the first portion 7a of the working channel 7 is 0.2 mm. An opening 7c of the working channel 7 has a diameter of 1 mm.

The camera assembly 6 and the working channel 7 are positioned bottom-to-top.

Especially if no working channel is present, such as in FIG 4a, the housing end wall 8e and circumferential wall 8g do not comprise any holes or openings. If the tip part assembly 5 includes a working channel 7, the working channel opening 7c is provided in the end wall 8e, 8e', 8e" such as seen in FIGs 4b, 4e and 4f. Positioning a working channel 7 in an opening 7c in the end wall 8e, 8e', 8e" does not carry the same risk of adhesive flowing out at a periphery of the opening 7c since adhesive residuals at a working channel opening 7c are not as disruptive as is the case for adhesive residuals at the camera assembly 6, where adhesive residuals may impact vision of the camera module.

At least during the method step where the adhesive 80 is filled into the housing 8, 8', 8", the housing 8, 8', 8" is positioned so that the proximal open end 8a faces upwards. This may also be the case in the method step where the adhesive 80 is allowed to harden.

During the method step of filling the spacing with an adhesive 80, an entire free volume of the spacing is filled up with the adhesive 80 up until an upper adhesive level 81.

Turning to FIGs. 5a - 5c it can be seen that the tip part assembly 5 further includes LEDs 9 for illuminating a target. The LEDs are positioned in the spacing before filling adhesive 80 into the spacing. The LEDs 9 are substantially embedded in the adhesive 80.

As seen in FIG. 5c The tip part assembly 5 may also include one or more light guides 50 for guiding light from respective LED(s) 9 to a distal end surface of the end wall 8e, 8e', 8e" of the housing thereof 8, 8', 8". The light guides shown are attached to a camera holder 10. It is also possible for one or more light guides 50 to be in one piece, potentially moulded in one piece with, the housing 8, 8', 8" of the tip part assembly 6. In the assembled position, the light guides 50 extend from the distal end 5b of the tip part assembly 5 to the respective set of LEDs 9. The light guides 50 are made from a transparent material and comprise a portion for abutting the camera assembly 6.

If no light guide 50 is present, the LED 9 may be positioned adjacent a proximal end surface of the end wall 8e, 8e', 8e", in which case the adhesive 80 is not present between the front surface of the LED 9 and the proximal end of the light guide 50, or between the front surface of the LED 9 and the proximal surface of the end wall 8e, 8e', 8e". The light guide 50 may be at least partly embedded in the adhesive 80 in the manufactured tip part assembly 5. The light guide 50 may include a light shield, in particular on a surface of the light guide 50 facing the camera assembly 6. The light shield may be provided as a layer of color or a material cladding with material having a low refraction index on said surface.

During manufacture of the tip part assembly 5, the camera assembly 6 and the LEDs 9 are first attached to respective holders, camera holder 10 and LED holder 90, where the interconnected holders 10, 90 and camera assembly 6 are then positioned in the housing 8, 8', 8" before filling in the adhesive 80.

### List of references

The following is a list of reference numerals used throughout this specification.
- 1: endoscope
- 11: monitor
- 12: cable socket
- 13: monitor cable
- 2: handle
- 21: control lever
- 3: insertion tube
- 3a: proximal end
- 3b: distal end
- 4: bending section
- 41: distal end segment
- 42: intermediate segment
- 43: proximal segment
- 5: tip part assembly
- 5a: tip part assembly proximal end
- 5b: tip part assembly distal end
- 50: light guide
- 51: camera holder
- 6: camera assembly
- 6a: camera assembly proximal end
- 6b: camera assembly distal end
- 60: camera module
- 60a: camera module proximal end
- 60c: camera module housing
- 60d: camera module connecting surface
- 7: working channel
- 7a: first working channel portion
- 7b: second working channel portion
- 7c: working channel hole or opening
- 70: circuit board
- 8: housing
- 8': housing
- 8‴: housing
- 8a: housing proximal end
- 8b: housing distal end
- 8c: outer surface
- 8e: housing end wall
- 8e': housing end wall
- 8e": housing end wall
- 8f: window
- 8g: circumferential wall
- 80: liquid adhesive
- 81: adhesive level
- 9: light-emitting diode (LED)
- 90: LED holder
- 10: Camera holder
- L: longitudinal direction
- PD: proximal-distal direction
- 6a: camera assembly proximal end
- 6b: camera assembly distal end
- 60: camera module
- 60a: camera module proximal end
- 60c: camera module housing
- 60d: camera module connecting surface
- 7: working channel
- 7a: first working channel portion
- 7b: second working channel portion
- 7c: working channel hole or opening
- 70: circuit board
- 8: housing
- 8': housing
- 8‴: housing
- 8a: housing proximal end
- 8b: housing distal end
- 8c: outer surface
- 8e: housing end wall
- 8e': housing end wall
- 8e": housing end wall
- 8f: window
- 8g: circumferential wall
- 80: liquid adhesive
- 81: adhesive level
- 9: light-emitting diode (LED)
- 90: LED holder
- 10: Camera holder
- L: longitudinal direction
- PD: proximal-distal direction

## Claims

1. A method of manufacture of a tip part assembly (5) for an endoscope (1), the tip part assembly (5) comprising a tip part and having a proximal end (3a) and a distal end (3b), the method comprising the steps of:
a) providing a camera assembly (6) of the tip part, the camera assembly (6) including a camera module (60), the camera assembly (6) having a distal end (6b) and a proximal end (6a) opposite the distal end (6b),
b) providing a cup-shaped housing (8, 8') having an open proximal end (8a), the housing (8, 8') further having a distal end (8b) positioned oppositely from the proximal end (8a) and defining a distal end (5b) of the manufactured tip part assembly (5), the housing (8, 8') further comprising a circumferential wall (8g) extending between the proximal and distal ends (8a, 8b) of the housing (8, 8') and a distal end wall (8e, 8e') positioned at the distal end (8b) of the housing (8, 8'), the circumferential wall (8g) and the distal end wall (8e, 8e') enclosing a spacing,
c) inserting the camera assembly (5) through the open proximal end (8a) of the housing (8, 8') so that the camera assembly (5) is at least partly positioned within the spacing,
d) subsequent to step c), filling a liquid adhesive (80) into the spacing through the open proximal end (8a) of the housing (8, 8') so that the camera assembly (5) is at least partly embedded in the adhesive (80), and so that the adhesive (80) substantially fills out all spacing volumes around the camera assembly (5),
e) subsequent to step d), allowing or causing the adhesive (80) to harden, whereby the adhesive (80) attaches the housing (8, 8') and the camera assembly (5) to each other, **characterized in that** in step c) a distal surface of a distal lens of the camera assembly (5) is positioned abutting a proximal surface of the housing distal end wall (8e, 8e').

2. A method according to claim 1, wherein the tip part assembly (5) further comprises a bending section (4) and the steps of:
f) providing the bending section (4), the bending section (4) having a distal end segment (41), and
g) subsequent to step e), adjoining the distal end segment (41) of the bending section (4) and the proximal open end (8a) of the housing.

3. A method according to claim 1 or 2, wherein the camera assembly (5) and potentially one or more LEDs (9) are first attached to a holder, the holder and camera assembly (5) and potentially LED(s) then being positioned in the housing before filling in the adhesive (80).

4. A method according to any one of the previous claims, wherein at least during step e) the housing is positioned so that the proximal open end (8a) faces upwards.

5. A method according to any one of the previous claims, wherein an entire free volume of the spacing is substantially filled up with the adhesive (80) up until an upper adhesive level (81).

6. A method according to any one of the previous claims, wherein an outer diameter of the circumferential wall (8g) of the housing is less than 3.3 mm.

7. A method according to any one of the previous claims, wherein the adhesive (80) substantially fills out all spacing volumes around the camera assembly (5), except for camera assembly holder(s) (10) and the distal end (6b) or front surface of the camera assembly (5) or the distal lens thereof, and wherein the adhesive (80) substantially fills out all spacing volume around potential LED(s) (9), except for LED holder(s) (90) and a distal end or front surface of such LED(s) (9).

8. A method according to any one of the previous claims, wherein the circumferential wall (8g) and/or the end wall of the housing is transparent.

9. A method according to any one of the previous claims, wherein the tip part assembly (5) further includes one or more light guides (50) and/or LED lenses in one piece with housing.

10. A method according to any one of the previous claims, wherein step c) includes moulding the housing in a two-component moulding process, wherein the distal end wall of the housing is moulded in a first material, which is transparent, and the circumferential wall (8g) is moulded in a second, different material, which is non-transparent.

11. A tip part assembly (5) for an endoscope (1), the tip part assembly (5) comprising a tip part and having a proximal end (3a) and a distal end (3b), the tip part assembly (5) comprising:
- a tip part including a cup-shaped housing (8, 8') and a camera assembly (6) positioned at least partly within a spacing of the housing (8, 8'), the camera assembly (6) being attached or adhered to the housing (8, 8') by means of a hardened adhesive (80) positioned within the spacing, the camera assembly (6) being at least partly embedded in the adhesive (80) and the adhesive (80) substantially filling out all spacing volumes around the camera assembly (6),
- wherein the camera assembly (6) includes a camera module (60), the camera assembly (6) having a distal end (6b) and a proximal end (6a) opposite the distal end (6b),
- wherein the housing (8, 8') has an open proximal end (8a) and a distal end (8b) positioned oppositely from the proximal end (8a), the distal end (8b) of the housing (8, 8') defining a distal end (5b) of the tip part assembly (5), the housing (8, 8') further comprising a circumferential wall (8g) extending between the proximal and distal ends (8a, 8b) of the housing (8, 8') and an end wall (8e, 8e') positioned at the distal end (8b) of the housing (8, 8'), the circumferential wall (8g) and the distal end wall (8e, 8e') enclosing the spacing,
- wherein the adhesive (80) and the housing (8, 8') are not moulded in one piece with each other,
- **characterized in that**
- a distal surface of a distal lens of the camera assembly (6) is positioned abutting a proximal surface of the housing distal end wall (8e, 8e').

12. A tip part assembly (5) according to claim 11, further comprising:
- a bending section (4) having a distal end segment (41), and
- wherein the distal end (41) of the bending section (4) and the proximal open end (8a) of the housing are adjoined to each other.

13. A tip part assembly (5) according to claim 11 or 12, wherein an entire volume of the spacing not preoccupied by the camera assembly (6) and potential further components positioned in the spacing is substantially filled up with the adhesive (80) up until an upper adhesive level (81), the upper adhesive level (81) being a level of the adhesive (80) towards the proximal end (8a) of the tip part assembly (5).

14. A tip part assembly (5) according to any one of the claims 11 to 13, wherein an outer diameter of the circumferential wall (8g) of the housing is less than 3.3 mm.

15. A tip part assembly (5) according to any one of claims 11 to 14, wherein the adhesive (80) substantially fills out all spacing volumes around the camera assembly (5), except for camera assembly holder(s) (10) and the distal end or the front surface of the camera assembly (5) or the distal lens thereof, and wherein the adhesive (80) substantially fills out all spacing volume around potential LED(s) (9), except for LED holder(s) (90) and a distal end or front surface of such LED(s) (9).

16. A tip part assembly (5) according to any one of the claims 11 to 15, wherein the circumferential wall (8g) and/or the end wall of the housing is transparent.

17. An endoscope (1) comprising a tip part assembly (5) manufactured according to any one of the claims 1 to 10 or a tip part assembly (5) according to any one of the claims 11 to 16.

## Patentansprüche

1. Verfahren zur Herstellung einer Spitzenteilanordnung (5) für ein Endoskop (1), wobei die Spitzenteilanordnung (5) ein Spitzenteil mit einem proximalen Ende (3a) und einem distalen Ende (3b) umfasst, wobei das Verfahren die folgenden Schritte umfasst:
a) Bereitstellen einer Kameraanordnung (6) des Spitzenteils, wobei die Kameraanordnung (6) ein Kameramodul (60) aufweist, wobei die Kameraanordnung (6) ein distales Ende (6b) und ein proximales Ende (6a) gegenüber dem distalen Ende (6b) hat,
b) Bereitstellen eines becherförmigen Gehäuses (8, 8') mit einem offenen proximalen Ende (8a), wobei das Gehäuse (8, 8') ferner ein distales Ende (8b) hat, das gegenüber dem proximalen Ende (8a) positioniert ist und ein distales Ende (5b) der hergestellten Spitzenteilanordnung (5) definiert, wobei das Gehäuse (8, 8') ferner eine Umfangswand (8g) umfasst, die sich zwischen dem proximalen und dem distalen Ende (8a, 8b) des Gehäuses (8, 8') erstreckt, und eine distale Endwand (8e, 8e'), die an dem distalen Ende (8b) des Gehäuses (8, 8') positioniert ist, wobei die Umfangswand (8g) und die distale Endwand (8e, 8e') einen Raum umschließen,
c) Einsetzen der Kameraanordnung (5) durch das offene proximale Ende (8a) des Gehäuses (8, 8'), sodass die Kameraanordnung (5) mindestens teilweise in dem Raum positioniert ist,
d) nach Schritt c) Einfüllen eines Flüssigklebers (80) in den Raum über das offene proximale Ende (8a) des Gehäuses (8, 8') derart, dass die Kameraanordnung (5) mindestens teilweise in den Kleber (80) eingebettet ist und derart, dass der Kleber (80) im Wesentlichen alle Zwischenräume rund um die Kameraanordnung (5) ausfüllt,
e) nach Schritt d) Ermöglichen oder Bewirken, dass der Kleber (80) härtet, wodurch der Kleber (80) das Gehäuse (8, 8') und die Kameraanordnung (5) aneinander befestigt, **dadurch gekennzeichnet, dass** in Schritt c) eine distale Oberfläche einer distalen Linse der Kameraanordnung (5) angrenzend an eine proximale Oberfläche der distalen Endwand (8e, 8e') des Gehäuses positioniert ist.

2. Verfahren nach Anspruch 1, wobei die Spitzenteilanordnung (5) ferner einen Biegeabschnitt (4) umfasst und die Schritte:
f) Bereitstellen des Biegeabschnitts (4), wobei der Biegeabschnitt (4) ein distales Endsegment (41) hat, und
g) nach Schritt e) Aneinanderfügen des distalen Endsegments (41) des Biegeabschnitts (4) und des proximalen offenen Endes (8a) des Gehäuses.

3. Verfahren nach Anspruch 1 oder 2, wobei die Kameraanordnung (5) und potenziell eine oder mehrere LEDs (9) zuerst an eine Halterung angebracht werden, wobei die Halterung und die Kameraanordnung (5) und die potenziellen LED(s) dann vor dem Einfüllen des Klebers (80) in dem Gehäuse positioniert werden.

4. Verfahren nach einem der vorhergehenden Ansprüche, wobei mindestens während Schritt e) das Gehäuse derart positioniert ist, dass das proximale offene Ende (8a) nach oben zeigt.

5. Verfahren nach einem der vorhergehenden Ansprüche, wobei ein völlig freies Volumen des Zwischenraums im Wesentlichen bis zu einer oberen Kleberfüllhöhe (81) mit dem Kleber (80) gefüllt wird.

6. Verfahren nach einem der vorhergehenden Ansprüche, wobei ein Außendurchmesser der Umfangswand (8g) des Gehäuses geringer ist als 3,3 mm.

7. Verfahren nach einem der vorhergehenden Ansprüche, wobei der Kleber (80) im Wesentlichen alle Zwischenräume rund um die Kameraanordnung (5) ausfüllt, mit Ausnahme der Kameraanordnungshalterung(en) (10) und des distalen Endes (6b) oder der Vorderseite der Kameraanordnung (5) oder der distalen Linse davon, und wobei der Kleber (80) im Wesentlichen alle Zwischenräume rund um die potenziellen LED(s) (9) ausfüllt, mit Ausnahme der LED-Halterung(en) (90) und einem distalen Ende oder einer Vorderseite solcher LED (s) (9) .

8. Verfahren nach einem der vorhergehenden Ansprüche, wobei die Umfangswand (8g) und/oder die Endwand des Gehäuses transparent ist.

9. Verfahren nach einem der vorhergehenden Ansprüche, wobei die Spitzenteilanordnung (5) ferner eine oder mehrere Lichtführungen (50) und/oder LED-Linsen einstückig mit dem Gehäuse aufweist.

10. Verfahren nach einem der vorhergehenden Ansprüche, wobei
Schritt c) das Gehäuse in einem Zweikomponentenformverfahren formt, wobei die distale Endwand des Gehäuses in einem ersten Material geformt wird, das transparent ist, und die Umfangswand (8g) in einem zweiten, anderen Material geformt wird, das nicht transparent ist.

11. Spitzenteilanordnung (5) für ein Endoskop (1), wobei die Spitzenteilanordnung (5) ein Spitzenteil mit einem proximalen Ende (3a) und einem distalen Ende (3b) umfasst, wobei die Spitzenteilanordnung (5) umfasst:
- ein Spitzenteil, das ein becherförmiges Gehäuse (8, 8') und eine Kameraanordnung (6) aufweist, die mindestens teilweise in einem Zwischenraum des Gehäuses (8, 8') positioniert ist, wobei die Kameraanordnung (6) an dem Gehäuse (8, 8') angebracht oder daran angeklebt ist mithilfe eines gehärteten Klebers (80), der in dem Zwischenraum positioniert ist, wobei die Kameraanordnung (6) mindestens teilweise in den Kleber (80) eingebettet ist und der Kleber (80) im Wesentlichen alle Zwischenräume rund um die Kameraanordnung (6) ausfüllt,
- wobei die Kameraanordnung (6) ein Kameramodul (60) aufweist, die Kameraanordnung (6) ein distales Ende (6b) und ein proximales Ende (6a) gegenüber dem distalen Ende (6b) hat,
- wobei das Gehäuse (8, 8') ein offenes proximales Ende (8a) und ein distales Ende (8b) hat, das gegenüber dem proximalen Ende (8a) positioniert ist, wobei das distale Ende (8b) des Gehäuses (8, 8') ein distales Ende (5b) der hergestellten Spitzenteilanordnung (5) definiert, wobei das Gehäuse (8, 8') ferner eine Umfangswand (8g) umfasst, die sich zwischen dem proximalen und dem distalen Ende (8a, 8b) des Gehäuses (8, 8') erstreckt, und eine Endwand (8e, 8e'), die an dem distalen Ende (8b) des Gehäuses (8, 8') positioniert ist, wobei die Umfangswand (8g) und die distale Endwand (8e, 8e') den Raum umschließen,
- wobei der Kleber (80) und das Gehäuse (8, 8') nicht einstückig miteinander geformt sind,
**dadurch gekennzeichnet, dass**
- eine distale Oberfläche einer distalen Linse der Kameraanordnung (6) angrenzend an eine proximale Oberfläche der distalen Endwand des Gehäuses (8e, 8e') positioniert ist.

12. Spitzenteilanordnung (5) nach Anspruch 11, ferner umfassend:
- einen Biegeabschnitt (4) mit einem distalen Endsegment (41), und
- wobei das distale Ende (41) des Biegeabschnitts (4) und das proximale offene Ende (8a) des Gehäuses aneinandergrenzen.

13. Spitzenteilanordnung (5) nach Anspruch 11 oder 12, wobei ein vollständiges Volumen des Zwischenraumes, der nicht von der Kameraanordnung (6) und potenziellen weiteren Komponenten, die in dem Zwischenraum positioniert sind, besetzt ist, im Wesentlichen bis zu einer oberen Kleberfüllhöhe (81) mit dem Kleber (80) aufgefüllt wird, wobei die obere Kleberfüllhöhe (81) eine Füllhöhe des Klebers (80) in Richtung des proximalen Endes (8a) der Spitzenteilanordnung (5) ist.

14. Spitzenteilanordnung (5) nach einem der Ansprüche 11 bis 13, wobei ein Außendurchmesser der Umfangswand (8g) des Gehäuses geringer als 3,3 mm ist.

15. Spitzenteilanordnung (5) nach einem der Ansprüche 11 bis 14, wobei der Kleber (80) im Wesentlichen alle Zwischenräume rund um die Kameraanordnung (5) ausfüllt, mit Ausnahme der Kameraanordnungshalterung(en) (10) und des distalen Endes oder der Vorderseite der Kameraanordnung (5) oder der distalen Linse davon, und wobei der Kleber (80) im Wesentlichen alle Zwischenräume rund um potenzielle LED(s) (9) ausfüllt, mit Ausnahme der LED-Halterung(en) (90) und eines distalen Endes oder einer Vorderseite solcher LED(s) (9) .

16. Spitzenteilanordnung (5) nach einem der Ansprüche 11 bis 15, wobei die Umfangswand (8g) und/oder die Endwand des Gehäuses transparent ist.

17. Endoskop (1), umfassend eine Spitzenteilanordnung (5), die gemäß einem der Ansprüche 1 bis 10 hergestellt wurde, oder Spitzenteilanordnung (5) nach einem der Ansprüche 11 bis 16.

## Revendications

1. Procédé de fabrication d'un ensemble partie d'embout (5) pour un endoscope (1), l'ensemble partie d'embout (5) comprenant une partie d'embout et ayant une extrémité proximale (3a) et une extrémité distale (3b), le procédé comprenant les étapes de :
a) fourniture d'un ensemble caméra (6) de la partie d'embout, l'ensemble caméra (6) comprenant un module de caméra (60), l'ensemble caméra (6) ayant une extrémité distale (6b) et une extrémité proximale (6a) opposée à l'extrémité distale (6b),
b) fourniture d'un boîtier en forme de coupelle (8, 8') ayant une extrémité proximale ouverte (8a), le boîtier (8, 8') ayant en outre une extrémité distale (8b) positionnée à l'opposé de l'extrémité proximale (8a) et définissant une extrémité distale (5b) de l'ensemble partie d'embout (5) fabriqué, le boîtier (8, 8') comprenant en outre une paroi circonférentielle (8g) s'étendant entre les extrémités proximale et distale (8a, 8b) du boîtier (8, 8') et une paroi d'extrémité distale (8e, 8e') positionnée à l'extrémité distale (8b) du boîtier (8, 8'), la paroi circonférentielle (8g) et la paroi d'extrémité distale (8e, 8e') renfermant un espacement,
c) l'insertion de l'ensemble caméra (5) à travers l'extrémité proximale ouverte (8a) du boîtier (8, 8') de sorte que l'ensemble caméra (5) soit au moins partiellement positionné à l'intérieur de l'espacement,
d) après l'étape c), le remplissage de l'espacement avec un adhésif liquide (80) à travers l'extrémité proximale ouverte (8a) du boîtier (8, 8') de sorte que l'ensemble caméra (5) soit au moins partiellement inclus dans l'adhésif (80), et de sorte que l'adhésif (80) remplisse sensiblement tous les volumes de l'espacement autour de l'ensemble caméra (5),
e) après l'étape d), le fait de laisser l'adhésif (80) durcir ou de l'amener à durcir, moyennant quoi l'adhésif (80) fixe le boîtier (8, 8') et l'ensemble caméra (5) l'un à l'autre, **caractérisé en ce que**, à l'étape c), une surface distale d'une lentille distale de l'ensemble caméra (5) est positionnée en butée contre une surface proximale de la paroi d'extrémité distale (8e, 8e') du boîtier.

2. Procédé selon la revendication 1, dans lequel l'ensemble partie d'embout (5) comprend en outre une section de courbure (4) et les étapes de :
f) fourniture de la section de courbure (4), la section de courbure (4) ayant un segment d'extrémité distale (41), et
g) après l'étape e), accollement du segment d'extrémité distale (41) de la section de courbure (4) à l'extrémité ouverte proximale (8a) du boîtier.

3. Procédé selon la revendication 1 ou 2, dans lequel l'ensemble caméra (5) et potentiellement une ou plusieurs DEL (9) sont d'abord fixés à un support, le support et l'ensemble caméra (5) et potentiellement la ou les DEL étant ensuite positionnés dans le boîtier avant le remplissage avec l'adhésif (80).

4. Procédé selon l'une quelconque des revendications précédentes, dans lequel, au moins au cours de l'étape e), le boîtier est positionné de sorte que l'extrémité ouverte proximale (8a) soit orientée vers le haut.

5. Procédé selon l'une quelconque des revendications précédentes, dans lequel un volume libre entier de l'espacement est sensiblement rempli par l'adhésif (80) jusqu'à un niveau supérieur d'adhésif (81).

6. Procédé selon l'une quelconque des revendications précédentes, dans lequel un diamètre externe de la paroi circonférentielle (8g) du boîtier est inférieur à 3,3 mm.

7. Procédé selon l'une quelconque des revendications précédentes, dans lequel l'adhésif (80) remplit sensiblement tous les volumes d'espacement autour de l'ensemble caméra (5), à l'exception du ou des supports d'ensemble caméra (10) et de l'extrémité distale (6b) ou de la surface avant de l'ensemble caméra (5) ou de sa lentille distale, et dans lequel l'adhésif (80) remplit sensiblement tout le volume d'espacement autour d'une ou plusieurs DEL potentielles (9), à l'exception d'un ou plusieurs supports de DEL (90) et d'une extrémité distale ou d'une surface avant de telles une ou plusieurs DEL (9) .

8. Procédé selon l'une quelconque des revendications précédentes, dans lequel la paroi circonférentielle (8g) et/ou la paroi d'extrémité du boîtier sont transparentes.

9. Procédé selon l'une quelconque des revendications précédentes, dans lequel l'ensemble partie d'embout (5) comprend en outre un ou plusieurs guides de lumière (50) et/ou lentilles de DEL d'un seul tenant avec le boîtier.

10. Procédé selon l'une quelconque des revendications précédentes, dans lequel l'étape c) comprend le moulage du boîtier dans un processus de moulage à deux composants, dans lequel la paroi d'extrémité distale du boîtier est moulée dans un premier matériau, qui est transparent, et la paroi circonférentielle (8g) est moulée dans un second matériau différent qui est non transparent.

11. Ensemble partie d'embout (5) pour un endoscope (1), l'ensemble partie d'embout (5) comprenant une partie d'embout et ayant une extrémité proximale (3a) et une extrémité distale (3b), l'ensemble partie d'embout (5) comprenant :
- une partie d'embout comportant un boîtier en forme de coupelle (8, 8') et un ensemble caméra (6) positionné au moins en partie à l'intérieur d'un espacement du boîtier (8, 8'), l'ensemble caméra (6) étant fixé ou mis à adhérer au boîtier (8, 8') au moyen d'un adhésif durci (80) positionné à l'intérieur de l'espacement, l'ensemble caméra (6) étant au moins en partie inclus dans l'adhésif (80) et l'adhésif (80) remplissant sensiblement tous les volumes d'espacement autour de l'ensemble caméra (6),
- dans lequel l'ensemble caméra (6) comporte un module de caméra (60), l'ensemble caméra (6) ayant une extrémité distale (6b) et une extrémité proximale (6a) opposée à l'extrémité distale (6b),
- dans lequel le boîtier (8, 8') présente une extrémité proximale ouverte (8a) et une extrémité distale (8b) positionnée à l'opposé de l'extrémité proximale (8a), l'extrémité distale (8b) du boîtier (8, 8') définissant une extrémité distale (5b) de l'ensemble partie d'embout (5), le boîtier (8, 8') comprenant en outre une paroi circonférentielle (8g) s'étendant entre les extrémités proximale et distale (8a, 8b) du boîtier (8, 8') et une paroi d'extrémité (8e, 8e') positionnée à l'extrémité distale (8b) du boîtier (8, 8'), la paroi circonférentielle (8g) et la paroi d'extrémité distale (8e, 8e') enfermant l'espacement,
- dans lequel l'adhésif (80) et le boîtier (8, 8') ne sont pas moulés d'un seul tenant l'un avec l'autre,
- **caractérisé en ce que**
- une surface distale d'une lentille distale de l'ensemble caméra (6) est positionnée en butée contre une surface proximale de la paroi d'extrémité distale (8e, 8e') du boîtier.

12. Ensemble partie d'embout (5) selon la revendication 11, comprenant en outre :
- une section de courbure (4) ayant un segment d'extrémité distale (41), et
- dans lequel l'extrémité distale (41) du segment de courbure (4) et l'extrémité ouverte proximale (8a) du boîtier sont accolées l'une à l'autre.

13. Ensemble partie d'embout (5) selon la revendication 11 ou 12, dans lequel un volume entier de l'espacement non préalablement occupé par l'ensemble caméra (6) et d'autres composants potentiels positionnés dans l'espacement est sensiblement rempli par l'adhésif (80) jusqu'à un niveau d'adhésif supérieur (81), le niveau d'adhésif supérieur (81) étant un niveau de l'adhésif (80) vers l'extrémité proximale (8a) de l'ensemble partie d'embout (5).

14. Ensemble partie d'embout (5) selon l'une quelconque des revendications 11 à 13, dans lequel un diamètre externe de la paroi circonférentielle (8g) du boîtier est inférieur à 3,3 mm.

15. Ensemble partie d'embout (5) selon l'une quelconque des revendications 11 à 14, dans lequel l'adhésif (80) remplit sensiblement tous les volumes d'espacement autour de l'ensemble caméra (5), à l'exception du ou des supports d'ensemble caméra (10) et de l'extrémité distale ou de la surface avant de l'ensemble caméra (5) ou de sa lentille distale, et dans lequel l'adhésif (80) remplit sensiblement tous les volumes d'espacement autour d'une ou plusieurs DEL potentielles (9), à l'exception du ou des supports de DEL (90) et d'une extrémité distale ou de la surface avant de telles une ou plusieurs DEL (9).

16. Ensemble partie d'embout (5) selon l'une quelconque des revendications 11 à 15, dans lequel la paroi circonférentielle (8g) et/ou la paroi d'extrémité du boîtier sont transparentes.

17. Endoscope (1) comprenant un ensemble partie d'embout (5) fabriqué selon l'une quelconque des revendications 1 à 10 ou un ensemble partie d'embout (5) selon l'une quelconque des revendications 11 à 16.
